# EUROPÄISCHE PATENTSCHRIFT

(11) **EP 1 443 852 B1**
(45) Veröffentlichungstag und Bekanntmachung des Hinweises auf die Patenterteilung: **16.07.2008**
(21) Anmeldenummer: 02774539.7
(22) Anmeldetag: 28.08.2002
(51) Int. Cl.: A61B 3/10

(54) **VERFAHREN UND VORRICHTUNG ZUR MESSUNG DES DYNAMISCHEN VERHALTENS DES AUGES**
METHOD AND DEVICE FOR MEASURING THE DYNAMIC BEHAVIOR OF AN OPTICAL SYSTEM
PROCEDE ET DISPOSITIF POUR MESURER LE COMPORTEMENT DYNAMIQUE D'UN SYSTEME OPTIQUE

(30) Priorität: 07.11.2001 DE 10154194
(43) Veröffentlichungstag der Anmeldung: 11.08.2004
(73) Patentinhaber: Carl Zeiss Meditec AG, 07745 Jena (DE)
(72) Erfinder: VOGELSANG, Hartmut, 07743 Jena (DE); BERGT, Michael, 07745 Jena (DE); DICK, Manfred, 07926 Gefell (DE); MÄUSEZAHL, Holger, 07745 Jena (DE); SCHRÖDER, Eckhard, 90542 Eckental (DE)
(74) Vertreter: DTS München
(86) Internationale Anmeldenummer: PCT/EP2002/009590
(87) Internationale Veröffentlichungsnummer: WO 2003/039356

(56) Entgegenhaltungen:
- WO-A-00/19885
- US-A- 6 155 684
- NAVARRO R ET AL: "PHASE PLATES FOR WAVE-ABERRATION COMPENSATION IN THE HUMAN EYE" OPTICS LETTERS, OPTICAL SOCIETY OF AMERICA, WASHINGTON, US, Bd. 25, Nr. 4, 15. Februar 2000 (2000-02-15), Seiten 236-238, XP000947124 ISSN: 0146-9592
- HUANG J ET AL: "Dynamic modeling and identification of an adaptive optics system" CONTROL APPLICATIONS, 1995., PROCEEDINGS OF THE 4TH IEEE CONFERENCE ON ALBANY, NY, USA 28-29 SEPT. 1995, NEW YORK, NY, USA,IEEE, US, 28. September 1995 (1995-09-28), Seiten 456-463, XP010207488 ISBN: 0-7803-2550-8
- HOFER H ET AL: "DYNAMICS OF THE EYE'S WAVE ABERRATION" JOURNAL OF THE OPTICAL SOCIETY OF AMERICA - A, OPTICAL SOCIETY OF AMERICA, WASHINGTON, US, Bd. 18, Nr. 3, März 2001 (2001-03), Seiten 497-506, XP001041247 ISSN: 1084-7529

## Beschreibung

Die Erfindung betrifft ein Verfahren und eine Vorrichtung zur Messung des dynamischen Verhaltens eines optischen Systems.

Die Analyse optischer Wellenfronten von abbildenden Systemen und Lasersystemen hat eine zunehmende Bedeutung erlangt, da dies Ausgangspunkt für die Qualitätssteigerung dieser Systeme ist. Mit der Verfügbarkeit kommerzieller Shack-Hartmann-Sensoren (z. B. SCLA-series, WavefrontSciences, http://wavefrontsciences.com) kann man.Aberrationen sehr genau erfassen und in Form von Zernike-Polynomen verschiedener Ordnungen oder alternativer Darstellungen klassifizieren.

Auch andere Aberrometer nach dem Tscherning-Prinzip, nach Abbe oder das Tracey-Aberrometer (ray-tracing) oder Systeme nach dem Skiaskop-Prinzip erlauben die Erfassung der höheren Aberrationen.

Systeme wie z. B. Shack-Hartmann-Sensoren, die mit einem CCD-Chip zur Speicherung der optisch relevanten Information ausgestattet sind, ermöglichen eine Datenakquirierung mit Videobildfrequenzen vorzunehmen und deshalb dynamische Abläufe ausreichend schnell aufzuzeichnen.

Bekannt sind Methoden, über die normale sphärische und zylindrische Korrektur der Abbildungsfehler hinausgehend, auch die höheren Aberrationen ab dritter Ordnung gemäß Seidel bzw. Zernike-Klassifizierung zu korrigieren. Dazu verwendet man z. B. adaptive Optiken, die als deformierbare Spiegel in Reflexion wirken oder Flüssigkristalloptiken, die in Transmission arbeiten. Diese adaptiven Optiken sind technologisch aufwendig und z. Z. noch nicht in allen Konsequenzen ausgereift. Sie erreichen z. Z. flächenhafte Auflösungen von typischerweise einigen Quadratmillimetern und werden bereits unter Laborbedingungen eingesetzt, um Wellenfronten in Rückkopplungsverfahren zwischen Wellenfrontmessung und adaptivem Element zu beeinflussen (siehe dazu Fernández, E. J. Iglesias, I., Artal, P. "Closed Loop Adaptive Optics in the Human Eye", Optics Letters, Vol. 26, No. 10, May 15, 2001). Diese Systeme sind bisher nicht eingesetzt worden, um neben der reinen Korrektur von Aberrationen hinausgehend echte Dynamikstudien bei Variation verschiedenster Sehbedingungen vorzunehmen.

Des Weiteren wurden insbesondere für ophtalmologische Anwendungen Methoden aufgezeigt, durch das optische System des Auges deformierte Wellenfronten auf einen Idealwert hin zu korrigieren, indem Aberrationen höherer Ordnung integral berücksichtigt werden (siehe dazu Optics Letters, Vol. 25 No. 4/February 15, 2000, 236 - 238, AWACS-Asclepion Wavefront Aberration Correction Simulator - Firmenunterlagen zur Präsentation auf der ESCRS in Brüssel, September 2000 und AAO in Dallas, Oktober 2000).

Dokument WO 00/19885 beschreibt eine Vorrichtung zur Messung der Refraktionsaberration des Auges.

Dokument US 6,155,684 befasst sich mit einem Verfahren und einer Vorrichtung zur Präkompensierung der refraktiven Eigenschaften des menschlichen Auges mit einer adaptiven optischen Regelkreissteuerung.

Der Artikel "Phase Plates for Wave-Aberration Compensation in the Human Eye" von Navarro R., et al (XP000947124) beschreibt ein Verfahren zur Herstellung von Phasenplatten zur Kompensierung von Wellenaberrationen im menschlichen Auge.

Der Artikel "Dynamic modeling and identification of an adaptive optics system" von Huan J., et al (XP010207488) betrifft einen neuen Ansatz zum Modellieren und Identifizieren von adaptiven optischen Systemen.

Der Artikel "Dynamics of the Eye's Wave Aberration" von HOFER H., et al (XP001041247) beschreibt einen Echtzeit-Hartmann-Shack-Wellenfrontsensor.

Der Artikel "Dynamic Retinal Image Reconstruction of the Human Eye" von C. Chao beschreibt Abberationen, die mittels eines Hartmann-Shack-Wellenfrontsensors ausgewertet werden.

Es ist daher Aufgabe der vorliegenden Erfindung, ein Verfahren, eine Vorrichtung sowie eine Anordnung bereitzustellen, mit denen das dynamische Verhalten eines optischen Systems objektiv erfassbar ist.

Dieses Problem wird durch ein Verfahren zur Messung des dynamischen Verhaltens eines Auges gemäß Anspruch 1 gelöst.

Das dynamische Verhalten des optischen Systems sind insbesondere Anpassungsvorgänge an veränderte Sehbedingungen, beispielsweise eine Akkomodation oder eine Blendenverstellung (Adaption). Das optische System kann ein Auge, beispielsweise ein menschliches Auge, ein künstliches Auge oder eine beliebige, künstliche Vorrichtung sein. Der Reiz kann grundsätzlich beliebiger Natur sein. Die Reaktion ist das dem Reiz nachfolgende dynamische Verhalten des optischen Systems. Die Wellenfrontanalyse kann beispielsweise mit einem Aberrometer, insbesondere mittels Shack-/Hartmann-Sensoren, aber auch mit Aberrometern nach dem Tscherning-Prinzip, nach Abbe, mit einem Ray-Tracing-Aberrometer oder System nach dem Siaskop-Prinzip durchgeführt werden. Bei der Akkomodation kann insbesondere der jeweils aktuelle Fokus des optischen Systems und dessen zeitlicher Verlauf erfasst werden. Ebenso können unterschiedliche Blendeneinstellungen bezüglich Ihres zeitlichen Verlaufs erfasst werden. Das dynamische Verhalten kann dabei beispielsweise auch unter Einfluß von Medikamenten untersucht werden.

In einer Ausgestaltung des Verfahrens ist vorgesehen, dass die Reize optische und/oder mechanische und/oder elektrische und/oder chemische Reize sind. Veränderliche optische Reize können beispielsweise in Form von aktiven steuerbaren Lichtquellen, beleuchteten Darstellungen oder dergleichen bewirkt werden. Insbesondere kann der die Abbildungsschärfe und/oder der Objektabstand und/oder dessen Fokus und/oder die Intensität des optischen Reizes verändert werden, sodass eine Akkomodation bzw. eine Blendenverstellung (Adaption) des optischen Systems provoziert werden kann. Ebenso kann eine Aberration auch höherer Ordnung, z.B. in Form einer entsprechend "deformierten" Objektwellenfront als optischer Reiz verwendet werden. Veränderliche mechanische Reize können beispielsweise als Luftzug durch ein Gebläse erzeugt werden. Veränderliche chemische Reize sind z.B. durch Rauch oder das Einbringen einer Flüssigkeit, eines Gases oder eines Aerosols realisierbar. Auch die Verabreichung als Medikament ist möglich. Veränderliche elektrische Reize sind unmittelbar durch an dem Auge oder im Bereich des Auges applizierte Elektroden oder durch induktive oder kapazitive Einkoppelung eines elektrischen Signals möglich. Die genannten Reize können jeweils einzeln oder beliebig miteinander kombinierbar aufgebracht werden und sowohl abrupt als auch kontinuierlich verändert werden.

In einer Ausgestaltung des Verfahrens ist vorgesehen, dass das zu messende optische System ein menschliches Auge ist. Das Verfahren zielt darauf ab, bei Stimulierung des einzelnen Auges oder des Augensystems beim Sehprozess eine gezielte Erregung und damit einhergehende Beeinflussung der Augenparameter herbeizuführen. Die mit den Beeinflussungen verbundenen Veränderungen der Augenparameter verändern unmittelbar die Abbildungseigenschaften des Auges und sind damit beispielsweise durch eine synchron zur Erregung getriggerte Wellenfrontmessung zugänglich. Mit Hilfe dieses Verfahrens können unterschiedlichste Effekte untersucht und gemessen werden. Beispielsweise kann die Zeitabhängigkeit und Geschwindigkeit der Akkommodation und der Akkommodationsfähigkeit, die Adaption und Adaptionsfähigkeit oder -geschwindigkeit des Auges unter Einflüssen wie z. B. Aberration, Beleuchtung, Medikamentation oder psychischer Einflüsse untersucht werden. Das dynamische Kurz- und Langzeitverhalten von Kontaktlinsen, beispielsweise Rutschen oder dergleichen, kann bezüglich der Veränderungen der Aberration durch Tragen von Kontaktlinsen untersucht werden. Das dynamische Verhalten von Intraokularlinsen (IOL), akkommodationsfähiger Intraokularlinsen und die Wechselwirkung des residualen Ziliarkörpers auf Intraokularlinsen sowie deren Passung und Bewegung und ggf. induzierter Akkommodation kann erfaßt werden. Des Weiteren sind Zusammenhänge zwischen dem physischen Sehen und der Gehirnleistung, die u. U. objektive Rückschlüsse auf Krankheitsbilder wie Kopfschmerzen durch Überanstrengung aufdecken helfen können, möglich. So können beispielsweise Blendwirkungen und andere zeitlich veränderliche Eindrücke im Zusammenhang mit Ermüdungserscheinungen beim Autofahren gemessen werden. Die optische dynamische Korrektur kann beispielsweise berufsgruppenbezogen, also auf bestimmte Anforderungen an das Sehvermögen, untersucht werden.

In einer Ausgestaltung des Verfahrens ist vorgesehen, dass die Stimulation des zu messenden Auges mit dem Aberrometer synchronisiert wird. Auf diese Weise ist es möglich, die gemessenen Adaptionsvorgänge des Auges unmittelbar dem jeweiligen Reiz und dessen zeitlichem Verlauf zuzuordnen. Die Synchronisierung kann dabei z.B. zeitlich oder bezüglich der Intensität zwischen dem jeweiligen Reiz und der Messung der Aberrometrie erfolgen

Bei der Durchführung des Verfahrens kann eines oder beide menschlichen Augen zu einer Reaktion stimuliert werden, ebenso kann eines oder beide menschlichen Augen gemessen werden. Aufgrund der gewonnenen stimulierten, dynamischen Messergebnisse können für das jeweils gemessene spezielle Auge optimierte/gemittelte Werte zur statischen/stationären Korrektur der Wellenfront abgeleitet werden. Damit kann man dem Auge für sein spezielles Aktionsspektrum eine optimale Wellenfrontkorrektur bereitstellen. Außerdem kann man für spezielle Sehvorgänge maßgeschneiderte Lösungen bereitstellen. Als Beispiele seien das Nachtsehen, geschwindigkeitsoptimierte Akkomodation oder das Nah- bzw. Fernsehen genannt.

Das eingangs genannte Problem wird auch durch eine Vorrichtung zur Messung des dynamischen Verhaltens eines optischen Systems, insbesondere eines menschlichen Auges gelöst, die eine Stimulationseinheit und ein Aberrometer umfasst. Mit der Stimulationseinheit können gezielt Anpassungsvorgänge auf äußere Reize des zu untersuchenden optischen Systems ausgelöst werden. Die Stimulationseinheit ist dazu so ausgelegt, dass diese äußere Reize auf das optische System ausüben kann. Als äußere Reize kommen prinzipiell alle physikalischen oder chemischen Effekte oder Mittel in Betracht, die eine Anpassungsreaktion des optischen Systems hervorrufen. Die Stimulationseinheit kann abrupt und/oder kontinuierlich auf das optische System einwirken und die entweder vor dem ungemessenen optischen System oder dem zu messenden optischen System angeordnet sein. Als Aberrometer werden hier allgemein Vorrichtungen zur Wellenfrontmessung oder zur Messung der Aberration verstanden. Dies können sowohl Vorrichtungen mit elektronischer Datenerfassung als auch manuell bedienbare Vorrichtungen sein.

Eine besonders einfache und zudem automatisierbare Auswertung der optischen Daten wird ermöglicht, wenn das Aberrometer eine Vorrichtung zur Wellenfrontanalyse umfasst. Die Vorrichtung zur Wellenfrontanalyse kann z. B. ein Shack-Hartmann-Sensor sein, der mit einem CCD-Chip zur Speicherung der optisch relevanten Information ausgestattet ist.

Die Messergebnisse und damit das Ergebnis des dynamischen Anpassungsvorganges können mittels Datenübergabe in eine Softwareanwendung grafisch visualisiert werden.

Besonders vorteilhaft ist es, wenn die Stimulationseinheit einen optischen und/oder mechanischen und/oder elektrischen und/oder chemischen Reiz auslösen kann. Insbesondere kann ein optischer Reiz so ausgelegt sein, dass eine Akkomodation oder Blendenvorstellung des optischen Systems provoziert wird. Auf diese Weise können unterschiedliche Reize auf das Auge ausgeübt werden und so auch Alltagssituationen wie z. B. Zugluft oder durch Rauch ausgelöste chemische Reize oder dergleichen simuliert werden.

Die Stimulationseinheit kann vor dem an dem Aberrometer vorbei schauenden Auge angeordnet sein, alternativ kann z.B. der Strahlengang der Stimulationseinheit in das Aberrometer eingespiegelt sein. Bei der erst genannten Ausführungsform der Vorrichtung kann das nicht zu untersuchende Auge durch Reize stimuliert werden, bei der zweitgenannten Ausführungsform der Vorrichtung kann das zu untersuchende Auge direkt durch äußere Reize stimuliert werden.

Alternativ kann die Stimulationseinheit in das Aberrometer integriert sein. In diesem Fall wird in einem gemeinsamen Gehäuse der Strahlengang der Stimulationseinheit unmittelbar in den des Aberrometers eingekoppelt. Dies ermöglicht eine sehr kompakte Bauweise.

Die Vorrichtung kann sowohl für monokulares als auch für binokulares Sehen ausgeführt sein. Ebenso kann die Stimulation ein oder beide Augen betreffen. Es können daher beliebige Kombinationen der Messung eines Auges und der Stimulation eines Auges realisiert sein, beispielsweise Stimulation eines Auges und Messung des anderen Auges, Stimulation eines Auges und Messung des gleichen Auges, Stimulation eines Auges und Messung beider Augen, Stimulation beider Augen und Messung eines Auges oder beider Augen.

In einer bevorzugten Ausführungsform ist vorgesehen, dass die Stimulationseinheit ein Fixierungsobjekt umfasst. Dieses soll von dem Probanden fixiert werden, so dass eine definierte Fokussierung des Auges erreicht wird. Das Fixierungsobjekt ist eine von dem Probanden leicht erkennbare bildliche Darstellung oder ein definierter Lichtfleck. Bevorzugt kommt hier ein fein strukturiertes Bild oder eine aus mehreren Elementen, die beispielsweise farblich abgestuft sein können, zusammengesetzte Lichtquelle in Betracht.

Vorteilhaft ist es, wenn das Fixierungsobjekt einen Lichtreiz abgeben kann, der in seiner Intensität und/oder Fokussierung veränderbar ist. Das Auge, das das Fixierungsobjekt fokussiert, kann auf diese Weise unmittelbar stimuliert werden. Die Änderung der Intensität kann beispielsweise durch eine Veränderung der Leuchtstärke bei Lichtquellen als aktiven Elementen oder durch Veränderung der Beleuchtungsstärke bei passiven Elementen wie einer beleuchteten Graphik erreicht werden. Eine Änderung der Fokussierung ist beispielsweise durch das Verfahren des Fixierungsobjekt selbst oder durch eine Veränderung vorgeschalteter Linsen möglich.

Das Fixierungsobjekt ist bevorzugt eine beleuchtete Grafik. Es handelt sich dabei um ein Objekt, das von dem Probanden leicht und sicher erkannt und fixiert werden kann und zudem einfach zu realisieren ist.

Die Vorrichtung kann mindestens eine in den Strahlengang der Stimulationseinheit und/oder des Aberrometers einbringbare Phasenplatte umfassen. Vorteilhaft wird ein Satz von Phasenplatten verwendet, welche nach den unterschiedlichen Zernike-Polynomen mit abgestuften Amplituden sortiert sind und die ähnlich Probierlinsen beim bekannten Phoropter vor dem zu untersuchenden System positioniert werden können. Als Phasenplatten können zum Beispiel transparente Glas- oder Kunststoffplatten benutzt werden, deren Oberfläche so strukturiert ist, dass einer durchlaufenden Lichtwelle definierte Aberrationen beispielsweise einem einzelnen Zernike-Term entsprechend aufgeprägt werden. In einer Wechselvorrichtung, vorzugsweise einem Wechselrevolver, sind vorzugsweise Platten einer Ordnung der Zernike-Koeffizienten mit unterschiedlicher Amplitude angeordnet. Durch zentrierte Anordnungen mehrerer solcher Wechselvorrichtungen hintereinander ist es möglich, unterschiedliche Phasenplatten in die optische Sehachse des zu untersuchenden optischen Systems einzuschwenken. Auf diese Weise kann auf der optischen Sehachse des zu untersuchenden, optischen Systems eine fein abstufbare Kombination von Abbildungsfehlern höherer Ordnung eingebracht werden. Mit dieser Anordnung können insbesondere Sehfehler, die auf höherer Aberration beruhen, subjektiv nach Erreichen eines angepassten, stationären Zustandes beispielsweise des menschlichen Auges bewertet werden. Die Bewertung erfolgt beispielsweise mit einer Zeitskala, z.B. nach einigen Sekunden der Anpassung.

Die Vorrichtung kann zur Auswertung der unterschiedlich stimulierten dynamischen Messdaten des Auges eine Software enthalten, die einen optimalen oder gemittelten Wert der Wellenfront bereitstellt, der zur statischen/stationären Korrektur der optischen Sehfehler bei den bekannten Korrekturverfahren (Brille, CL, IOL, LASIK, PRK, ...) verwendet wird.

Das eingangs genannte Problem wird auch durch eine Anordnung zur Messung des dynamischen Verhaltens eines optischen Systems, bevorzugt eines Auges, umfassend ein Aberrometer sowie eine Stimulationseinheit gelöst. Bei dieser Anordnung wird eine Trennung der zuvor im Rahmen der Vorrichtung gemeinsam realisierten Elemente vorgenommen. Es kann daher auch eine herkömmliche Vorrichtung zur Messung der Aberration eines Auges mit einer eigenständigen Stimulationseinheit verwendet werden. Das eingangs genannte Problem wird auch durch die Verwendung einer Vorrichtung nach einem der auf eine Vorrichtung gerichteten Ansprüche zur Messung des dynamischen Verhaltens eines optischen Systems gelöst.

Vorteilhafte Ausgestaltungen der Erfindung werden weiter in den Zeichnungen erläutert. Dabei zeigen:
- Fig. 1: eine Prinzipskizze einer Vorrichtung zur Durchführung des Verfahrens;
- Fig. 2: eine erste Ausführungsform der erfindungsgemäßen Vorrichtung als Prinzipskizze;
- Fig. 3: eine zweite Ausführungsform der erfindungsgemäßen Vorrichtung als Prinzipskizze.

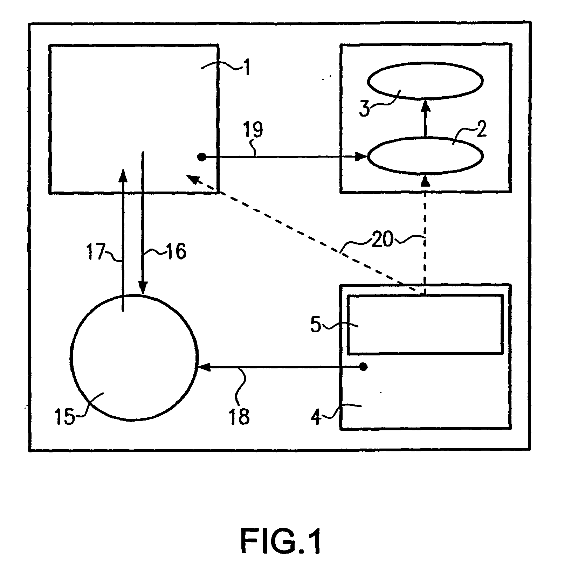

Zunächst wird auf Fig. 1 Bezug genommen. Diese zeigt die Prinzipskizze einer Vorrichtung sowie des damit zusammenhängenden Verfahrensablaufes zur dynamischen Stimulation und dynamischen Messung der Aberrometrie eines Auges 15. Dargestellt sind jeweils die einzelnen Komponenten in ihrem Wirkungszusammenhang. Die Vorrichtung umfasst ein Gerät zur Wellenfrontmessung/Aberrometrie des Auges 15, bei dem entweder beide Augen gleichzeitig oder jeweils ein Auge gemessen werden kann. Im letzten genannten Fall kann das gerade nicht gemessene Auge gegebenenfalls frei am Gerät vorbei blicken oder blickt ebenfalls in das Gerät. Der Einfachheit halber wird das Gerät zur Wellenfrontmessung/Aberrometrie eines Auges im Folgenden Aberrometer 1 genannt. Von dem Aberrometer 1 wird Messlicht 16 in das Auge 15 eingestrahhlt, welches Signallicht 17 zurückstrahlt. Messlicht 16 und Signallicht 17 sind durch Pfeile in Fig. 1 angedeutet. Die Vorrichtung umfasst des Weiteren eine Einrichtung zur dynamischen Datenakquirierung 2, die Rohdaten 19 des Aberrometers 1 entgegennimmt. Es kann sich dabei um gängige elektronische Vorrichtungen zur Messwerterfassung handeln. Diese Vorrichtung kann beispielsweise ein programmgesteuerter Rechner mit einer Software sein, die insbesondere sequenzielle Mess-Serien in Echtzeit akquirieren kann. Gegebenenfalls ist dafür eine Datenzwischenspeicherung in einem flüchtigen oder nicht flüchtigen Speicher z. B. eines Steuerungscomputers notwendig. Die Einrichtung umfasst des Weiteren eine Analysesoftware, welche die akquirierten Daten bzw. Datensätze analysieren kann, um die bestimmenden Parameter der Wellenfront (z. B. Zernike-, Taylor-Koeffizienten) zu berechnen. Die Messergebnisse und damit das Ergebnis des dynamischen Anpassungsvorganges können mittels einer Softwareanwendung grafisch visualisiert werden. Dazu dient ein Analysemodul 3, an das die ermittelten Daten übergeben werden. Die Fähigkeiten der dynamischen Datenakquirierung 2 sowie des Analysemodules 3 können so kombiniert werden, dass eine der Hardware angepasste und gegebenenfalls in der Akquirierungsrate reduzierte sequenzielle Echtzeitmessung mit simultaner Analyse stattfinden kann. Des Weiteren ist eine Stimulationseinheit 4 zur abrupten und/oder kontinuierlich veränderlichen optischen Einwirkung wie beispielsweise Aberration, Lichteinfluss, Objektabstand oder dergleichen vorhanden, die entweder vor dem ungemessenen Auge 15 oder dem zu messenden Auge 15 angeordnet ist und die mit der dynamischen Datenakquirierung 2 synchronisiert sein kann. Die Stimulation des Auges 15 ist in Fig. 1 durch einen Pfeil 18 angedeutet Eine Synchronisierungseinheit 5 dient der Synchronisierung zwischen Stimulationseinheit 4 und Aberrometer 1. Die Synchronisierungseinheit kann Synchronisierungsimpulse 20, in Fig. 1 durch gestrichelte Pfeile angedeutet, an das Aberrometer und/oder die Einrichtung zur dynamischen Datenaquirierung 2 abgeben.

Die Stimulationseinheit 4 kann auch als eigenständiges, separates Gerät realisiert sein und eingesetzt werden. Das dann vorliegende Gerät stellt eine Art Stimulationsphoropter dar, durch den der Proband z. B. eine Phasenplattenkorrektur mit oder ohne Variation anderer Sehparameter beurteilen kann.

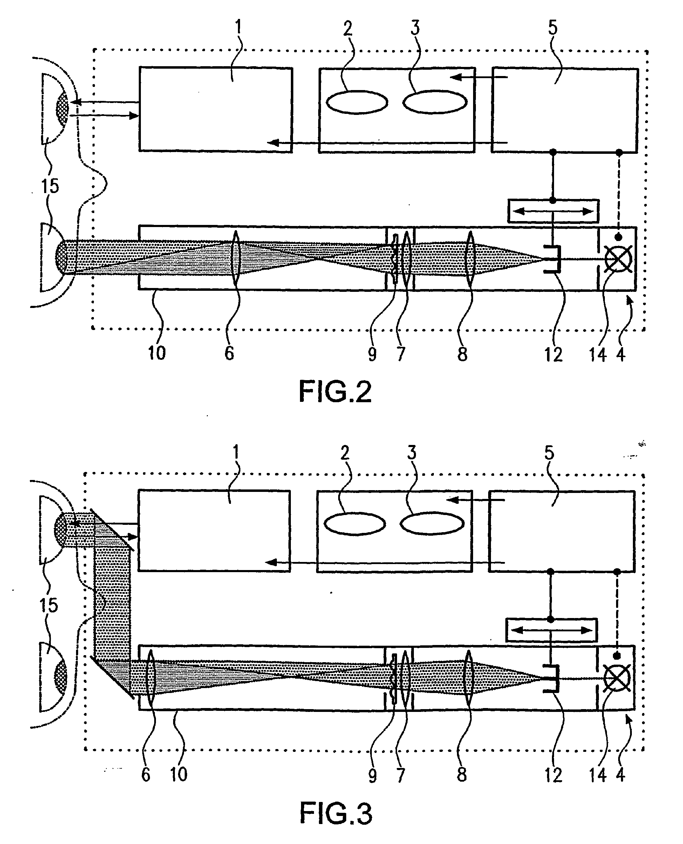

Die Fig. 2 und 3 zeigen Ausführungsformen der Vorrichtung. Die dargestellten Strahlverläufe durch eine erste Linse 6, eine zweite Linse 7 und eine dritte Linse 8 entsprechen nicht genau den realen Strahlverläufen bei Betrachtung im Rahmen der geometrischen Optik, sondern sollen nur zur Veranschaulichung dienen. Die konkrete Realisierung des optischen Konzeptes kann so erfolgen, dass die Nase des Patienten kein Hindernis darstellt. In den Fig. 2 und 3 ist der optische Aufbau so dargestellt, dass sich eine Phasenplatte 9 in einer zur Hornhautoberfläche oder Brillenkorrekturfläche konjugierten Ebene befindet. Durch Integration eines nicht dargestellten Mechanismus zum automatischen Wechsel der Phasenplatten 9, beispielsweise in Form eines Phasenplattenwechselrades oder dergleichen, können verschiedene Aberrationen bequem auch während oder zwischen Mess-Serien vorgenommen werden. Eine erste Blende 10 sowie eine zweite Blende 11 geben zusammen mit der Fixierung auf ein Fixierungsobjekt 12 die optische Achse vor. Gegebenenfalls können weitere Zieleinrichtungen vorgesehen werden, z.B. Flächenkreuze. Die Zentrierung kann darüber hinaus durch ein Videobild kontrolliert werden, das innerhalb der Vorrichtung der Fig. 2 und 3 beispielsweise durch Strahlteiler abgegriffen werden kann. Variierende Akkommodationszustände können durch Verfahren des Fixierungsobjektes entlang eines Verfahrweges 13 oder alternativ beispielsweise der dritten Linse 8 stimuliert werden. Die Adaption kann durch Einstellung der Beleuchtung des Fixierungsobjektes 12 durch eine Lichtquelle 14 beeinflusst werden. Zusätzlich oder alternativ kann eine direkte Einstellung der Helligkeit des Raumlichtes und/oder des Umgebungslichtes stattfinden. Diese Möglichkeit ist der Einfachheit halber nicht dargestellt. Die einzelnen Komponenten können elektrisch bzw. elektromotorisch angesteuert und angetrieben werden. Die Synchronisierung dieser Komponenten mit der Messung und Datenakquirierung kann dann z. B. bequem über die Abfrage programmierbarer Schnittstellen erfolgen.

Bei der Vorrichtung nach Fig. 2 wird ein Auge 15 gemessen und das jeweils andere Auge 15 stimuliert, bei der Vorrichtung nach Fig. 3 wird das zu messende Auge 15 gleichzeitig stimuliert. Zunächst wird der gemeinsame Aufbau in der Fig. 2 erläutert. Die Vorrichtung umfasst ein Aberrometer 1, es handelt sich dabei um ein Gerät zur Messung der Wellenfrontverformung durch das optische System des Auges und damit zur Bestimmung und Klassifizierung der Abbildungsfehler des Auges 15, dabei werden auch Abbildungsfehler höherer Ordnung umfasst. Das Aberrometer 1 ist gekoppelt mit einer Einrichtung zur dynamischen Datenakquirierung 2. Es handelt sich hier um ein Modul zur Ansteuerung des Aberrometers 1. Dieses Modul kann einen Wellenfrontmessvorgang auslösen. Ebenso können die gewonnenen Messdaten zwischengespeichert werden und aus den Messdaten sodann die gemessene Wellenfront rekonstruiert werden. Dabei können beispielsweise die Rohdaten des Videobildes des Sensors oder auch vollständig ausgewertete Wellenfrontparameter wie beispielsweise Zernike-Koeffizienten gespeichert werden. Messung und eventuelle Auswertung und Speicherung erfolgen mit Taktraten, die schneller als der zu untersuchende Anpassungsvorgang sind. Die Taktraten können beispielsweise im Bereich von 10 bis 100 Hz liegen.

Die Einrichtung zur dynamischen Datenakquirierung 2 ist mit einem Analysemodul 3 gekoppelt. Das Analysemodul 3 dient der Auswertung der Sensordaten und gegebenenfalls der Rekonstruktion und grafischen Visualisierung der gemessenen und gespeicherten Wellenfronten und ist weitgehend in Software realisierbar. Des Weiteren kann eine Parametrisierung der Wellenfront z. B. durch Entwicklung nach Zernike-Polynomen oder durch zonale Rekonstruktion durchgeführt werden. Der Analyseprozess kann in enger Kopplung mit der Einrichtung zur dynamischen Datenakquirierung 2 gekoppelt sein und den Analyseprozess teilweise oder vollständig vor der Zwischenspeicherung vornehmen.

Eine Stimulationseinheit 4 besteht im Wesentlichen aus einem optischen System, das dem zu untersuchenden oder dem freien Auge 15 ein zu fixierendes, detailliert strukturiertes Sehobjekt darbietet und das Auge 15 zur Fokussierung auf die Struktur des Objektes bewegt. Die Stimulationseinheit 4 kann optische Elemente wie z. B. Linsen oder Phasenplatten aufweisen, die die vom Sehobjekt ausgehende Wellenfront verformen, bevor sie ins Auge 15 gelangen. Dadurch kann das Auge 15, das bemüht ist, ein scharfes Bild vom Sehobjekt zu erhalten, zu Anpassungsreaktionen innerhalb des optischen Systems stimuliert werden. Bevorzugt können die abbildenden Eigenschaften der Stimulationseinheit 4 zeitlich variiert werden, um eine dynamische Reaktion des Auges 15 hervorzurufen. Die Stimulationseinheit 4 kann Informationen über Ihren momentanen Zustand an eine Synchronisationseinheit 5 mit der Wellenfrontmessung senden.

Die Synchronisationseinheit 5 ist ein Modul zur Synchronisation von dynamischen Veränderungen in der Stimulationseinheit 4 mit der Einrichtung zur dynamischen Datenakquirierung 2. Ziel ist es, die gemessenen Wellenfrontdaten mit den jeweiligen Zuständen der Synchronisationseinheit zu korrelieren.

Die in den Fig. 2 und 3 dargestellten Lösungen eines Aberrometers 1 können als dynamische Stimulations-Aberroskope bezeichnet werden. Hier ist zu beachten, dass das optische Konzept so vorgenommen werden kann, dass das ungemessene Auge nicht wie dargestellt in seinem Blick eingeschränkt werden muss, sondern frei blicken kann. Weitere Varianten benutzen zur Stimulation statt eines integrierten Fixierungsobjektes z. B. eine ruhende oder zur Abstandsvariation bewegliche Sehtafel und/oder einen Phasenplattenphoropter.

Die in Fig. 2 gezeigte Ausführungsform für eine Stimulationseinheit 4 ist auch als Verbesserung zum einfachen Phasenplattenphoropter zu sehen und kann in den verschiedenen Varianten z. B. mit einer Korrektur der Aberration und einer einfachen Sehtafel als Fixierungsobjekt usw. als alleinstehendes Gerät ausgelegt werden.

Für die gezielte Aufprägung von Aberrationen auf die Objektwellenfront kann wahlweise statt einer Phasenplatte und/oder der abbildenden Optik gemäß Fig. 2 und 3 auch ein adaptives optisches Element eingebracht werden. Die dabei stimulierten dynamischen Veränderungen der Abbildungseigenschaften des Auges 15 werden mit dem Aberrometer 1 in einer zeitlichen Sequenz dynamisch erfasst, die mit der Variation der Abbildungseigenschaften durch die adaptive Optik synchronisiert werden kann. Transmissionsbasierte adaptive Elemente wie z. B. Flüssigkristall-Phasenmodulatoren können z. B. statt der Phasenplatte in ähnlicher Weise in die Anordnung gemäß den Fig. 2 und 3 eingebaut werden.

Obwohl die Verwendung einer adaptiven Optik aufwendiger ist, da zusätzlich eine elektronische Ansteuerung der adaptiven Optik erforderlich wird, bieten sich Möglichkeiten der Stimulation, die Geräte mit Phasenplatten nicht oder nur schwer erlauben. Je nach Ansteuergeschwindigkeit der adaptiven optischen Elemente können beliebige Aberrationen der Objektwellenfront dynamisch verändert oder gezielt appliziert werden.

Die Stimulationseinheit 4 wird als optisches System ausgelegt, welches entweder vor dem frei vorbei schauenden Auge 15 platziert wird oder in den Strahlengang des mit dem Aberrometer untersuchten Auges eingespiegelt wird, wie dies in Fig. 3 dargestellt ist, oder die Stimulationseinheit 4 wird in das Aberrometer 1 integriert. Auch bei dieser letzten Ausführungsform sind wiederum zwei Varianten möglich, die Stimulationeinheit kann auf das oder die gemessenen Augen 15 einwirken oder die Stimulationseinheit 4 kann auf das ungemessene Auge 15 einwirken.

Bei der Stimulationseinheit selbst handelt es sich um ein optisches Gerät, bei dem ein Fixierungsobjekt vor das oder die gemessenen oder ungemessenen bzw. frei blickenden Augen platziert wird und auf dessen Zentrum das jeweilige Auge während der Untersuchung blicken und fokussieren soll. Die optische Wirkung bei der Stimulation kann entweder abrupt oder kontinuierlich moduliert werden, was z. B. durch Änderung der Entfernung, Leuchtdichte usw. des Fixierungsobjektes erreichbar ist und/oder durch Anbringen von Phasenplatten. Alle optisch wirksamen Modifikationen sind beliebig kombinierbar. Wird die optische Wirkung mit der dynamischen Messung des Auges 15 durch das Aberrometer 1 synchronisiert z. B. im Sinne einer Zeitsynchronisierung oder einer Beleuchtungssynchronisierung, so erhält man Ausführungsformen der Geräte entsprechend der Fig. 2 und 3. Die Wirkung muss nicht zwangsläufig synchronisiert werden. Informationen über die dynamischen Reaktionen des Auges bzw. der Augen können auch unsynchronisiert gemessen werden. Dazu kann z. B. über die Kenntnis der Datenakquirierungsfrequenz der Einrichtung zur dynamischen Datenakquirierung 2 eine zeitliche Zuordnung der Messwerte vorgenommen werden, diese können beispielsweise bei einer elektronischen Speicherung auf einem Datenträger oder dergleichen mit einem Zeitstempel versehen werden. Die Einrichtung zur dynamischen Datenakquirierung 2 und das Analysemodul 3 können auch so kombiniert werden, dass nicht nur eine dynamische Datenakquirierung, sondern gleichzeitig eine Hochgeschwindigkeitsanalyse der Daten erfolgt.

Das Fixierungsobjekt 12 kann z. B. durch eine beleuchtete Grafik mit hinreichend feiner Strukturierung realisiert werden, kann aber auch eine einfache, separat positionierte Sehtafel sein.

Die Abbildung des Fixierungsobjektes 12 kann durch Einschwenken von optischen Elementen wie Linsen erreicht werden und ggf. über ein Videosystem kontrolliert werden. Die Zentrierung auf eine vorgegebene Sehrichtung kann durch Blendensysteme optimiert werden. Eine automatische Positionierung z. B. erlaubt durch Variation von Abständen zwischen den optischen Komponenten und/oder dem Fixierungsobjekt gezielt Akkommodationszustände hervorzurufen. Eine geeignete Beleuchtungseinstellung des Fixierungsobjektes und/oder des Raumlichtes führt zu definierten Adaptionseinstellungen, die ebenfalls variabel gehalten werden können und zusätzliche Messparameter bilden.

Durch Einbringen speziell präparierter Phasenplatten mit definierter Oberflächentopografie in den Strahlengang der Stimulationseinheit 4 und/oder des Aberrometers 1 erlaubt die Vorrichtung die gezielte Applizierung von Aberrationen auf das oder die Augen 15. Die Phasenplatten können dazu in einem hier nicht dargestellten Wechselrevolver angeordnet sein und einzeln oder in Kombination in den Strahlengang der Stimulationseinheit 4 und/oder des Aberrometers 1 einbringbar sein.

Die zuvor dargestellte Vorrichtung und das damit ausführbare Verfahren dient der gezielten visuellen Stimulation eines biologischen oder künstlichen Auges 15 und zur Erfassung des damit verbundenen dynamischen Anpassungsvorgang des optischen Sehapparates durch Messen der Wellenfrontaberration. Die visuelle Stimulation, welche beim Blick in bzw. durch eine geeignete Apparatur hervorgerufen wird, führt zu einer Beeinflussung der Abbildungseigenschaften des Auges 15, welche gleichzeitig zeitsynchronisiert zur Stimulation in Echtzeit mit einem Wellenfrontanalysesystem bzw. einem Aberrometer 1 gemessen werden kann. Dies erlaubt völlig neue Diagnosemöglichkeiten, welche die Dynamik von Anpassungsvorgängen des Auges 15 zugänglich macht. Dazu werden Sehbedingungen wie Objektabstand und Helligkeit modifiziert und insbesondere gleichzeitig bestimmte Aberrationen gezielt korrigiert und/oder eingebracht. Damit kann z. B. der Einfluss bestimmter Aberrationsterme auf die Akkommodationsfähigkeit studiert werden, oder es kann untersucht werden, ob eine implantierbare Intraokularlinse vermöge des residualen Ziliarkörpers akkommodationsfähig ist und wie das gegebenenfalls optimal nutzbar wäre. Ein Vorteil der Erfindung liegt auch darin, subjektive Eindrücke bei der Bewertung von dynamischen Sehprozessen durch einen Probanden mit physikalisch objektiven Messdaten korrelieren zu können.

Mit der Vorrichtung und dem Verfahren ist es möglich, dynamische Änderungen der Abbildungseigenschaften des Auges zu stimulieren und deren zeitlichen Verlauf aufzuzeichnen. Mit Hilfe eines Systems von optischen Elementen können bestehende Abbildungsfehler auch höherer Ordnung zielgerichtet kompensiert und andere Abbildungsfehler gezielt zur Stimulation eingebracht werden, um die Einflüsse auf die Dynamik des optischen Systems des Auges 15 zu erfassen. Damit kann man verschiedenste dynamische Vorgänge wie z. B. während der Akkommodation oder Adaption in einer Bildfolge oder einem Film der Entwicklung der Wellenfrontaberrationen dokumentieren. Daraus lassen sich dynamische Parameter wie z. B. Anpassungsbereich, - zeiten, -geschwindigkeiten oder -beschleunigungen z. B. bei der Akkommodation oder der Adaption ableiten. Damit werden Rückschlüsse auf anatomische Parameter wie Elastizität der Augenlinse, die z. B. mit Fragestellungen der Wechselwirkung der Verformung von Augenlinse und Hornhaut und der Dynamik von Intraokularlinsen verbunden sein kann, oder auch das primäre Reaktionsvermögen des Auges objektiv messbar. Es können Grundzusammenhänge der Wirkung von Medikamentierung oder z.B. Ursache-Wirkungszusammenhänge von Krankheitsbildern wie z.B. Kopfschmerzen, Müdigkeit, Überanstrengung aufgedeckt werden.

Die beschriebene Erfindung bietet die Möglichkeit der objektiven dynamischen Messung der Abbildungseigenschaften des Auges bei gezielt stimulierten Anpassungsvorgängen unter vorgegebenen Randbedingungen.

### BEZUGSZEICHENLISTE

- 1: Aberrometer
- 2: Einrichtung zur dynamischen Datenaquirierung
- 3: Analysemodul
- 4: Stimulationseinheit
- 5: Synchronisierungseinheit
- 6: Erste Linse
- 7: Zweite Linse
- 8: Dritte Linse
- 9: Phasenplatte
- 10: Erste Blende
- 11: Zweite Blende
- 12: Fixierungsobjekt
- 13: Verfahrweg
- 14: Lichtquelle
- 15: Auge
- 16: Messlicht
- 17: Signallicht
- 18: Stimulation
- 19: Rohdaten
- 20: Synchronisierungsimpulse

## Patentansprüche

1. Verfahren zur Messung des dynamischen Verhaltens eines Auges (15), wobei das zu messende Auge (15) durch Reize zu einer Akkommodation und/oder Adaption auf diese Reize stimuliert wird, **dadurch gekennzeichnet, dass** der zeitliche Verlauf der Akkommodation und/oder Adaption auf diese Reize mittels einer Wellenfrontanalyse erfasst wird.

2. Verfahren nach Anspruch 1, wobei die Akkomodation und/oder Adaption dynamische Änderungen der Abbildungseigenschaften des zu messenden optischen Systems sind, insbesondere der zeitliche Verlauf von Fokus oder Blendeneinstellung sind.

3. Verfahren nach einem der vorhergehenden Ansprüche, **dadurch gekennzeichnet, dass** das zu messende Auge (15) ein menschliches Auge ist.

4. Verfahren nach einem der vorhergehenden Ansprüche, **dadurch gekennzeichnet, dass** die Reize mit einer Vorrichtung zur Wellenfrontanalyse synchronisiert werden.

5. Verfahren nach einem der vorhergehenden Ansprüche, **dadurch gekennzeichnet, dass** eines oder beide Augen (15) zu einer dynamischen Änderungen ihrer Abbildungseigenschaften stimuliert werden und dass eines oder beide Augen (15) gemessen werden.

6. Vorrichtung zur Messung des dynamischen Verhaltens eines Auges (15), wobei diese eine Stimulationseinheit (4) und ein Aberrometer (1) umfasst, und wobei die Stimulationseinheit (4) das Auge (15) durch Reize zu einer Akkommodation und/oder einer Adaption stimulieren kann, wobei die Vorrichtung weiter eine Synchronisierungseinheit (5) zur Synchronisierung zwischen Stimulationseinheit (4) und Aberrometer (1) umfasst, **dadurch gekennzeichnet, dass** das Aberrometer (1) eine Vorrichtung umfasst, mit welcher der zeitliche Verlauf der Akkomodation und/oder Adaption auf diese Reize mittels einer wellenfrontanalyse erfasst werden kann.

7. Vorrichtung nach Anspruch 6, wobei die Akkomodation und/oder Adaption dynamische Änderungen der Abbildungseigenschaften sind, insbesondere der zeitliche Verlauf von Fokus oder Blendeneinstellung sind.

8. Vorrichtung nach Ansprüche 5, **dadurch gekennzeichnet, dass** die Stimulationseinheit (4) einen optischen und/oder mechanischen und/oder elektrischen und/oder chemischen Reiz auslösen kann.

9. Vorrichtung nach einem der vorhergehenden Vorrichtungsansprüche, **dadurch gekennzeichnet, dass** eines oder beide Augen (15) zu einer dynamischen Änderungen ihrer Abbildungseigenschaften stimuliert werden können und dass eines oder beide Augen (15) gemessen werden können.

10. Vorrichtung nach Anspruch 9, **dadurch gekennzeichnet, dass** die Stimulationseinheit (4) vor dem an dem Aberrometer (1) vorbeischauenden Auge (15) angeordnet ist.

11. Vorrichtung nach Anspruch 9, **dadurch gekennzeichnet, dass** der Strahlengang der Stimulationseinheit (4) in das Aberrometer (1) eingespiegelt ist.

12. Vorrichtung nach Anspruch 9, **dadurch gekennzeichnet, dass** die Stimulationseinheit (4) in das Aberrometer (1) integriert ist.

13. Vorrichtung nach einem der Ansprüche 6 bis 12, **dadurch gekennzeichnet, dass** die Stimulationseinheit (4) ein Fixierungsobjekt (12) umfasst.

14. Vorrichtung nach Anspruch 13, **dadurch gekennzeichnet, dass** das Fixierungsobjekt (12) einen Lichtreiz abgeben kann, der in seiner Intensität und/oder Fokus veränderbar ist.

15. Vorrichtung nach einem der Ansprüche 13 oder 14, **dadurch gekennzeichnet, dass** das Fixierungsobjekt (12) eine beleuchtete Graphik ist.

16. Vorrichtung nach einem der Ansprüche 6 bis 15, **dadurch gekennzeichnet, dass** diese mindestens eine in den Strahlengang der Stimulationseinheit (4) und/oder des Aberrometers (1) einbringbare Phasenplatte umfasst.

## Claims

1. Method of measuring the dynamic behaviour of an eye (15), wherein the eye (15) to be measured is stimulated by stimuli to an accommodation and/or adaptation to these stimuli, **characterized in that** the temporal course of the accommodation and/or adaptation to these stimuli is recorded by means of a wavefront analysis.

2. Method according to claim 1, wherein the accommodation and/or adaptation are dynamic changes to the image-forming qualities of the optical system to be measured, in particular the temporal course of focus or diaphragm setting.

3. Method according to one of the previous claims, **characterized in that** the eye (15) to be measured is a human eye.

4. Method according to one of the previous claims, **characterized in that** the stimuli are synchronized with a device for the wavefront analysis.

5. Method according to one of the previous claims, **characterized in that** one or both eyes (15) are stimulated to dynamic changes to their image-forming qualities and that one or both eyes (15) are measured.

6. Device for measuring the dynamic behaviour of an eye (15), wherein this comprises a stimulation unit (4) and an aberrometer (1), and wherein the stimulation unit (4) can stimulate the eye (15) to an accommodation and/or an adaptation by stimuli, wherein the device also comprises a synchronization unit (5) for the synchronization between stimulation unit (4) and aberrometer (1), **characterized in that** the aberrometer (1) comprises a device with which the temporal course of the accommodation and/or adaptation to these stimuli can be recorded by means of a wavefront analysis.

7. Method according to claim 6, wherein the accommodation and/or adaptation are dynamic changes to the image-forming qualities, in particular the temporal course of focus or diaphragm setting.

8. Device according to claim 5, **characterized in that** the stimulation unit (4) can trigger an optical and/or mechanical and/or electrical and/or chemical stimulus.

9. Method according to one of the previous claims, **characterized in that** one or both eyes (15) can be stimulated to dynamic changes to their image-forming qualities and that one or both eyes (15) can be measured.

10. Device according to claim 9, **characterized in that** the stimulation unit (4) is arranged in front of the eye (15) travelling past the aberrometer (1).

11. Device according to claim 9, **characterized in that** the beam path of the stimulation unit (4) is reflected into the aberrometer (1).

12. Device according to claim 9, **characterized in that** the stimulation unit (4) is integrated in the aberrometer (1).

13. Device according to one of claims 6 to 12, **characterized in that** the stimulation unit (4) comprises a fixing object (12).

14. Device according to claim 13, **characterized in that** the fixing object (12) can emit a light stimulus, the intensity and/or focus of which can be changed.

15. Device according to one of claims 13 or 14, **characterized in that** the fixing object (12) is an illuminated graphic.

16. Device according to one of claims 6 to 15, **characterized in that** this comprises at least one phase plate which can be introduced into the beam path of the stimulation unit (4) and/or of the aberrometer (1).

## Revendications

1. Procédé pour mesurer le comportement dynamique d'un oeil (15), l'oeil (15) à mesurer étant stimulé par excitations pour obtenir une accommodation et/ou une adaptation à la suite de cette excitation, **caractérisé en ce que** la courbe dans le temps de l'accommodation et/ou de l'adaptation à la suite de cette excitation est réalisée à l'aide d'une analyse de front d'onde.

2. Procédé selon la revendication 1, l'accommodation et/ou l'adaptation étant des modifications dynamiques des propriétés d'image du système optique à mesurer, notamment de la courbe dans le temps de l'ouverture de foyer ou de diaphragme.

3. Procédé selon l'une quelconque des revendications précédentes, **caractérisé en ce que** l'oeil (15) à mesurer est un oeil humain.

4. Procédé selon l'une quelconque des revendications précédentes, **caractérisé en ce que** les excitations sont synchronisées avec un dispositif d'analyse de front d'onde.

5. Procédé selon l'une quelconque des revendications précédentes, **caractérisé en ce que** un oeil ou les deux yeux (15) sont stimulés pour modifier de façon dynamique leurs propriétés d'image et **en ce qu'**un oeil ou les deux yeux (15) sont mesurés.

6. Dispositif pour mesurer le comportement dynamique d'un oeil {15), celui-ci comprenant une unité de stimulation (4) et un dispositif de mesure d'aberration (1) et l'unité de stimulation (4) pouvant stimuler l'oeil (15) par excitation pour provoquer une accommodation et/ou une adaptation, le dispositif comprenant en outre une unité de synchronisation (5) pour réaliser la synchronisation entre l'unité de stimulation (4) et le dispositif de mesure d'aberration (1), **caractérisé en ce que** le dispositif de mesure d'aberration (1) comprend un dispositif permettant de déterminer la courbe dans le temps de l'accommodation et/ou de l'adaptation à la suite de ces excitations à l'aide d'une analyse de front d'onde.

7. Dispositif selon la revendication 6, l'accommodation et/ou l'adaptation étant des modifications dynamiques des propriétés d'image, notamment de la courbe dans le temps de l'ouverture de foyer ou de diaphragme.

8. Dispositif selon la revendication 5, **caractérisé en ce que** l'unité de stimulation (4) peut déclencher une excitation optique et/ou mécanique et/ou électrique et/ou chimique.

9. Dispositif selon l'une quelconque des revendications de dispositif précédentes, **caractérisé en ce qu'**un oeil ou les deux yeux (15) peuvent être stimulés pour modifier de façon dynamique leurs propriétés d'image et **en ce qu'**un oeil ou les deux yeux (15) peuvent être mesurés.

10. Dispositif selon la revendication 9, **caractérisé en ce que** l'unité de stimulation (4) est disposée avant l'oeil (15) regardant vers le dispositif de mesure d'aberration (1).

11. Dispositif selon la revendication 9, **caractérisé en ce que** le parcours optique de l'unité de stimulation (4) se reflète dans le dispositif de mesure d'aberration (1).

12. Dispositif selon la revendication 9, **caractérisé en ce que** l'unité de stimulation (4) est intégrée dans le dispositif de mesure d'aberration (1).

13. Dispositif selon l'une quelconque des revendications 6 à 12, **caractérisé en ce que** l'unité de stimulation (4) comprend un objet à fixer (12).

14. Dispositif selon la revendication 13, **caractérisé en ce que** l'objet à fixer (12) est entouré d'une excitation lumineuse dont l'intensité et/ou le foyer peuvent changer.

15. Dispositif selon l'une quelconque des revendications 13 ou 14, **caractérisé en ce que** l'objet à fixer (12) est une illustration éclairée.

16. Dispositif selon l'une quelconque des revendications 6 à 15, **caractérisé en ce que** cet au moins un parcours optique de l'unité de stimulation (4) et/ou du dispositif de mesure d'aberration (1) comprend une plaque de phases pouvant être insérée.
